# EUROPEAN PATENT SPECIFICATION

(11) **EP 3 805 406 B1**
(45) Date of publication and mention of the grant of the patent: **20.04.2022**
(21) Application number: 19202126.9
(22) Date of filing: 09.10.2019
(51) Int. Cl.: C12Q 1/6851, C12Q 1/686

(54) **DIGITAL AND QUANTITATIVE PCR MEASURING METHOD FOR NUCLEIC ACID SAMPLE**
DIGITALES UND QUANTITATIVES PCR-MESSVERFAHREN FÜR NUKLEINSÄUREPROBE
PROCÉDÉ DE MESURE QUANTITATIF NUMÉRIQUE DE RÉACTION EN CHAÎNE PAR POLYMÉRASE POUR ÉCHANTILLON D'ACIDE NUCLÉIQUE

(43) Date of publication of application: 14.04.2021
(73) Proprietor: Quark Biosciences Taiwan, Inc., Zhubei City, Hsinchu County 30261 (TW)
(72) Inventor: WEI, Cheng-Wey, 30261 Hsinchu County (TW); HUANG, Chang-Wei, 30261 Hsinchu County (TW)
(74) Representative: Becker Kurig & Partner Patentanwälte mbB

(56) References cited:
- WO-A1-2019/144907
- US-A1- 2018 258 465
- US-A1- 2019 256 896
- ZHOU SHUFANG ET AL: "A highly integrated real-time digital PCR device for accurate DNA quantitative analysis", BIOSENSORS AND BIOELECTRONICS, vol. 128, 11 January 2019 (2019-01-11), pages 151-158, XP085583621, ISSN: 0956-5663, DOI: 10.1016/J.BIOS.2018.12.055

## Description

### BACKGROUND OF THE INVENTION

### Field of the Invention

The invention relates to a digital and quantitative PCR measuring method for a nucleic acid sample, and more particularly, to a measuring method suitable for a nucleic acid sample to be tested having a plurality of nucleic acid targets with different concentration ranges of wide variations.

### Description of Related Art

The advantage of the existing digital PCR is that sample concentration can be directly measured without performing a calibration curve in the experimental detection. However, the disadvantage of inconvenient use exist in the practical application. The reason is that, digital PCR mainly utilizes the Poisson Distribution to estimate the sample concentration, and the sample concentration must not be too high in a way that all the detection reaction wells demonstrate positive responses. That is, if the sample concentration successfully measured by digital PCR is expected, the sample concentration must be kept low to a certain extent, and the sample must not be distributed to all of the detection reaction wells. In this way, when facing a sample of unknown concentration, it is necessary to use other methods to perform the preliminarily quantification and dilution first. As a result, the sample concentration falls within a concentration range applicable to digital PCR, so as to measure the sample concentration successfully by digital PCR. Therefore, the dynamic range of detection and operational convenience are both limited.

In general, in the nucleic acid sample to be tested, the nucleic acid targets may have wide concentration ranges, and this phenomenon is especially common in clinical samples. If the nucleic acid targets with higher concentration and lower concentration are simultaneously presented in the nucleic acid sample, the nucleic acid sample needs to be diluted for digital PCR measurement. As a result, the nucleic acid target with higher concentration falls within a concentration range applicable to digital PCR, so that the nucleic acid target with higher concentration can be measured by digital PCR. However, while diluting the nucleic acid sample, the nucleic acid target with higher concentration is able to fall within a concentration range applicable to the digital PCR, but the nucleic acid target with lower concentration is over-diluted and cannot be detected, resulting in the issue of reduced sensitivity. This situation often occurs in the detection of gene mutations in liquid biopsy samples. For example, when a wild-type gene with higher concentration and a mutation gene with lower concentration are simultaneously presented in the nucleic acid sample to be tested, the sensitivity of detection may be affected.

Currently on the market, the number of reaction well or droplet for detection is usually increased, so that there may be enough negative reaction wells after the high-concentration nucleic acid target is distributed, which meets the applicable conditions of digital PCR, and the copy number of the sample to be tested can be measured. However, increasing the number of reaction wells for detection increases the difficulty of the platform technique and enhances the cost and time of detection.

Based on the above, when detecting a nucleic acid target with higher concentration via digital PCR, the sample concentration can be successfully detected without increasing the number of reaction wells and diluting the sample to improve dynamic range and operational convenience, is an important topic requiring research.

WO 2019/144907 A1 discloses a detection instrument for a digital PCR, a quantitative detection method for a digital PCR, a quantitative analysis method for a digital PCR having different volume, a detection method for a digital PCR, a microsphere for a nucleic acid test, a preparation method for a microsphere for a nucleic acid test, a kit for a microsphere for a nucleic acid test, and a detection method for a high-throughput nucleic acid.

US 2018/258465 A1 discloses a device that can perform multiple independent digital PCRs with real time monitoring capability. The device comprises multiple PCR mini-reactors thermally coupled with its own temperature control element, a detection unit, and a motorized platform for holding and moving the PCR mini-reactors. The real time digital PCR device can simultaneously perform multiple digital PCRs.

ZHOU SHUFANG ET AL: "A highly integrated real-time digital PCR device for accurate DNA quantitative analysis", BIOSENSORS AND BIOELECTRONICS, vol. 128, pages 151-158 discloses a Raspberry Pi based, low-cost and highly integrated device to achieve real-time fluorescence detection for microfluidic digital array, termed real-time dPCR device. In the device, uniform thermocycler, streamlined real-time fluorescence imaging setup, and compact data processing system are all integrated to undergo on-chip dPCR amplification, real-time fluorescence detection, and data analysis.

### SUMMARY OF THE INVENTION

The invention is provided by the appended claims. The following disclosure serves a better understanding of the invention and provides a measuring method for a nucleic acid sample, having the characteristic of simultaneously performing real-time quantitative polymerase chain reaction (qPCR) and digital PCR. The measuring method of the invention can detect high-concentration and low-concentration nucleic acid targets simultaneously presented at one time, so as to expand the dynamic range of detection. This method is called digital and quantitative PCR, which has the dual function of digital PCR and quantitative PCR.

The measuring method for the nucleic acid sample of the invention includes the following steps. A testing plate having a plurality of reaction wells is provided, so as to perform a digital and quantitative real-time PCR on the nucleic acid sample. In particular, a copy number of the low-concentration nucleic acid target is directly quantified by the function of digital PCR. A Cq value of the high-concentration nucleic acid target is detected by the function of qPCR, and a converting step is performed to obtain a copy number of the high-concentration nucleic acid target in the nucleic acid sample.

In an embodiment of the invention, a PCR efficiency is obtained by a qPCR reaction curve. After that, the converting step is performed, including the following steps. 1 is added to the PCR efficiency as a base. The Cq value of the high-concentration nucleic acid target is subtracted from a qPCR Cq value of the low-concentration nucleic acid target to obtain ΔCq as an exponent. Next, the base and the exponent are used for a power operation, so as to obtain a copy number of a nucleic acid target for each of the reaction wells, which is multiplied by a total number of reaction wells in the digital PCR to obtain a total copy number of the high-concentration nucleic acid target.

In an embodiment of the invention, the nucleic acid sample contains more than one kind of nucleic acid targets, and a plurality of the nucleic acid targets has different concentration ranges.

In an embodiment of the invention, the digital and quantitative real-time PCR is performed using 64 or more reaction wells.

In an embodiment of the invention, the digital and quantitative real-time PCR is performed using 64 to 20000 reaction wells.

In an embodiment of the invention, after the converting step, a dynamic range is increased to 9 logs.

In an embodiment of the invention, a testing plate having 2500 experimental reaction wells is used. When the concentration of nucleic acid target is high (meaning all of the 2500 experimental reaction wells demonstrate positive responses to the nucleic acid target), the copy number of the nucleic acid target in the nucleic acid sample is higher than 10000.

In an embodiment of the invention, when the concentration of nucleic acid target is low (meaning not all of the experimental reaction wells demonstrate positive responses to the nucleic acid target), the copy number of the nucleic acid target in the nucleic acid sample can be directly measured.

In an embodiment of the invention, a testing plate having 2500 experimental reaction wells is used. When not all of the 2500 experimental reaction wells demonstrate positive responses to the nucleic acid target, the copy number of the nucleic acid target in the nucleic acid sample is 10000 or less.

Based on the above, the invention provides a measuring method for a nucleic acid sample (referred to as digital and quantitative PCR), wherein a copy number of the low-concentration nucleic acid target is directly quantified by the function of digital PCR. A Cq value of the high-concentration nucleic acid target is detected by the function of qPCR, and a converting step is performed to obtain a copy number of the high-concentration nucleic acid target. In this way, when the high-concentration nucleic acid target is detected, the sample concentration can be successfully detected without diluting the sample, so the dynamic range of detection and the operational convenience can be effectively improved.

In order to make the aforementioned features and advantages of the invention more comprehensible, embodiments accompanied with figures are described in detail below.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

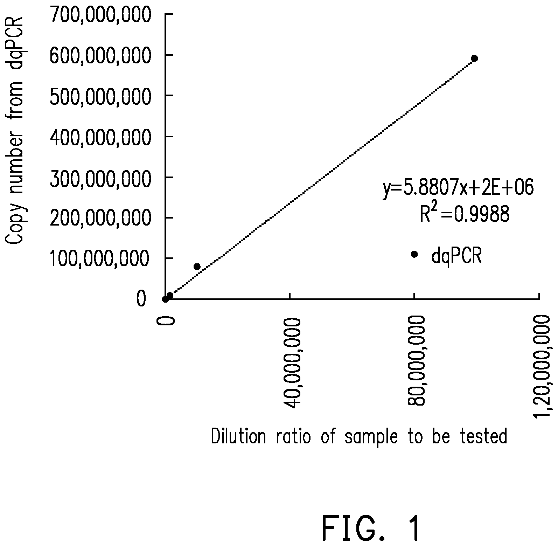

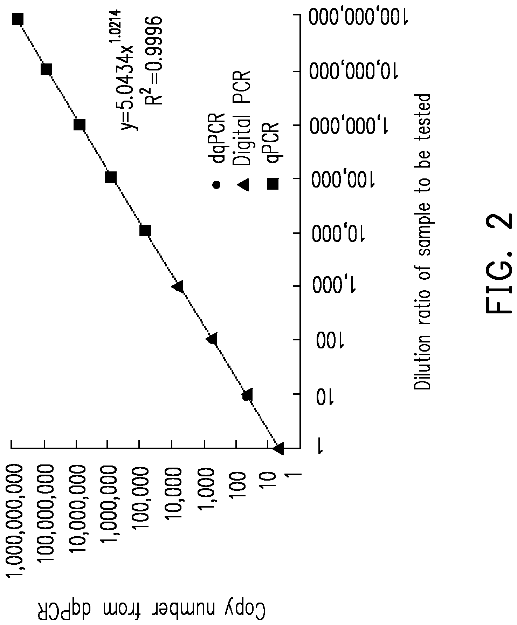

FIG. 1 and FIG. 2 are test results of a measuring method for a nucleic acid sample according to an embodiment of the invention, which illustrate how the invention simultaneously performs the dual functions of digital PCR and quantitative PCR, so as to achieve a dynamic range of 9 logs, and convert the qPCR Cq value of a high-concentration nucleic acid target to the copy number of the high-concentration nucleic acid target via a converting step.

### DESCRIPTION OF THE EMBODIMENTS

The invention provides a measuring method for a nucleic acid sample. In the following, the terminology used in the specification is first defined and explained.

"qPCR" or "real-time quantitative PCR" refers to an experimental method in which PCR is used to amplify and simultaneously quantify a target DNA. Quantification is performed using a variety of chemical substances for detection (including a fluorescent dye of SYBR^{®} green or a fluorescent reporter oligonucleotide probe of Taqman probe, etc.), and real-time quantification is performed on amplified DNA accumulated in the reaction after each amplification cycle.

"Digital PCR" is an absolute quantification technique for nucleic acid molecules. Compared to qPCR, digital PCR can directly measure the copy number of nucleic acid molecules. A sample is divided into tens to tens of thousands of parts and distributed to different reaction wells, and then PCR amplification is respectively performed on the nucleic acid target in each of the reaction wells. After the amplification is completed, the fluorescent signal of each of the reaction wells is analysed.

"Cq value" is the number of amplification cycles at which the fluorescence intensity is significantly increased during the qPCR operation process.

"PCR efficiency" refers to the increasing amount of nucleic acid after each PCR cycle. A good design usually results in an efficiency between 90% and 110%, which means that the amount of the nucleic acid may be increased by 90% to 110% after each additional PCR cycle. The method references a method of using the increasing amount of fluorescence intensity to measure the PCR efficiency in the literature (Biochem Biophys Res Commun. 2002 Jun 7; 294(2): 347-53), and the PCR efficiency is equal to (R_{Cq}-R_{Cq-1})/R_{Cq-1}, wherein R_{Cq} is the fluorescence intensity of the cycle of Cq, R_{Cq-1} is the fluorescence intensity of the cycle of Cq-1, and fluorescent background value needs to be deducted first from both R_{Cq} and R_{Cq-1}.

"Sample" refers to the nucleic acid sample being tested. For example, the sample may be a nucleic acid fragment (including DNA or RNA, etc.) extracted from a source such as blood, tissue, saliva, or the like. "Template" refers to a DNA or RNA or microRNA strand with a specific sequence, also referred to as a biomarker and can be detected via a qPCR reaction.

"Testing plate having a plurality of reaction wells" refers to a slide plate having a plurality of reaction wells, wherein each of the reaction wells is used to perform a dqPCR reaction.

"Dynamic range" usually refers to a linear dynamic range, which refers to one interval of the concentration range, within which the known sample concentration (e.g. known serial dilution ratio) and the measured sample concentration thereof show acceptable linearity (Huggett, Jim F., et al., Clinical Chemistry, 2013, 59(6), 892-902). The unit of dynamic range is usually expressed in logs.

The invention provides a measuring method for a nucleic acid sample. First, a testing plate having a plurality of reaction wells is provided to perform digital and quantitative PCR on a nucleic acid sample, which may contain one or more kinds of nucleic acid targets. When the nucleic acid sample contains more than one kinds of nucleic acid targets, the concentration range of each nucleic acid target may be different or even significantly different. The reaction wells in the testing plate are individually assigned to measure different types of nucleic acid templates having diverse concentration ranges at one time. In the present embodiment, for example, a digital and quantitative PCR reaction is performed using 64 or more reaction wells, and preferably, for example, a digital and quantitative PCR reaction is performed using 64 to 20000 reaction wells.

When all reaction wells demonstrate positive responses to the nucleic acid target, the concentration of the nucleic acid target is higher, and the copy number of the nucleic acid target in the nucleic acid sample is, for example, greater than 10000. In the existing digital PCR, for a nucleic acid target with higher concentration, dilution needs to be performed first. As a result, the sample concentration falls within a concentration range applicable to digital PCR, so that the sample concentration may be measured successfully by digital PCR. However, in the invention, a converting step is performed when all reaction wells demonstrate positive responses for the nucleic acid target, so as to obtain a copy number of the nucleic acid target with higher concentration in the nucleic acid sample. In this way, the sample concentration can be successfully detected without diluting the sample.

In the invention, a PCR efficiency is obtained by a qPCR reaction curve, and then the converting step is performed, including the following steps. 1 is added to the PCR efficiency as a base. The Cq value of the high-concentration nucleic acid target is subtracted from a qPCR Cq value of the low-concentration nucleic acid target to obtain ΔCq as an exponent. After that, the base and the exponent are used for a power operation, so as to obtain a copy number of a nucleic acid target for each reaction well, which is multiplied by a total number of reaction wells in the digital PCR to obtain a converted total copy number of the high-concentration nucleic acid target.

When not all experimental reaction wells demonstrate positive responses to the nucleic acid target, the concentration of this nucleic acid target is lower. The copy number of the nucleic acid target in the nucleic acid sample is, for example, 10000 or less, and the specific range of the copy number is, for example, 1 to 10000. Via the measuring method for the nucleic acid sample of the invention, the copy number of the nucleic acid target in the nucleic acid sample can be directly measured by quantitative PCR.

In the measuring method for the nucleic acid sample of the invention, the characteristics of real-time quantitative polymerase chain reaction (qPCR) and digital PCR can be combined. The dynamic range of digital PCR is 3 logs, and the dynamic range of qPCR is 6 logs. In the measuring method for the nucleic acid sample of the invention, the dynamic range can be increased to 9 logs by a dqPCR technique.

In order to measure the dynamic range of the detection, a commonly used method in literature (Huggett, Jim F., et al., Clinical Chemistry, 2013, 59(6), 892-902). is to prepare a sample to be tested and then to perform several times of consecutive serial dilutions on this sample, such as 5 times of consecutive 10-fold serial dilutions to obtain a total of 6 serially-diluted samples (100000X, 10000X, 1000X, 100X, 10X, 1X). After that, the 6 serially-diluted samples are detected to obtain the detected concentrations thereof, and then the linearity between the six samples with known dilution ratios and the respective detection concentrations thereof is calculated. If the linearity reaches a correlation coefficient R² greater than 0.98, it means that this detection may reach a dynamic range of 6 logs.

FIG. 1, FIG. 2 and Table 1 are detected results obtained by a measuring method for a nucleic acid sample according to an embodiment of the invention, which illustrate how the dual functions of digital PCR and quantitative PCR of the invention are simultaneously performed, so as to achieve a dynamic range of 9 logs (the correlation coefficient R² of the known serial dilution concentration and the detected concentration (copy number) measured by dqPCR is 0.99 or more), and the qPCR Cq value of the high-concentration nucleic acid target is converted by a converting step to the copy number of the high-concentration nucleic acid target.

The consecutive 10-fold diluted samples derived from the same sample are listed in Table 1 from top to bottom, wherein the top 5 dilution ratios are 100,000,000X to 10,000X. All reaction wells are positive after the PCR reaction and cannot be directly measured by digital PCR. All of the last 4 dilution ratios show some negative reaction wells after the reaction, and the copy number of the sample per well can be measured by digital PCR, which is multiplied by the reaction well number to obtain the total copy number. For example, when the sample dilution ratio is 100X, the copy number per well measured by digital PCR is 0.23, which is multiplied by a reaction well number of 2500 to obtain a total copy number of 576. When the sample dilution ratio is 10,000X or more, the Cq value of the sample can be measured by quantitative PCR, and the total copy number can be obtained by the conversion of the present method. For example, when the sample dilution ratio is 10,000X, the Cq value measured by quantitative PCR is 20.72. The Cq value of this sample at a single copy number is 25.66, and the PCR efficiency is 92%. 20.72 is subtracted from 25.66 to obtain 4.94. 1 is added to the efficiency of 0.92 to obtain 1.92, and 1.92 to the power of 4.94 is 24.97, which is the converted copy number per well and multiplied by the reaction well number of 2500 gives a converted total copy number of 62416. The nine samples are linearly regressed by the converted total copy number obtained by the present method and the sample dilution ratio. The correlation coefficients R² are 0.9988 (FIG. 1) and 0.9996 (FIG. 2), indicating that the present method is excellent in feasibility and linearity.

The data source of FIG. 1 and FIG. 2 is Table 1, the X coordinate axis of FIG. 1 is the sample dilution ratio of Table 1, and the Y axis of FIG. 1 is the total copy number of the sample to be tested of Table 1. In FIG. 1, since the maximum value of the Y axis is higher, the lower coordinate points may not be distinguished in the figure. In order to clearly show each coordinate in the figure, FIG. 1 are revised and shown as FIG. 2, in which the coordinate axes in FIG. 1 are revised to the form of logarithm with a base of 10 (log₁₀).

**Table 1**

| Sample dilution ratio | Well number | Copy number /well (from digital PCR) | Cq (from qPCR) | PCR efficiency | Base | Exponent | Converted copy number/well (base^ exponent) | Total copy number of sample to be tested | Measuring method |
|---|---|---|---|---|---|---|---|---|---|
| 100,000,000 X | 2500 | >3 | 6.67 | 0.92 | 1.92 | 18.99 | 235528.49 | 58882123 7 | qPCR |
| 10,000,000 X | 2500 | >3 | 9.76 | 0.92 | 1.92 | 15.90 | 31532.26 | 78830657 | qPCR |
| 1,000,000X | 2500 | >3 | 13.39 | 0.92 | 1.92 | 12.27 | 2968.89 | 7422227 | qPCR |
| 100,000X | 2500 | >3 | 17.01 | 0.92 | 1.92 | 8.65 | 280.63 | 701570 | qPCR |
| 10,000X | 2500 | >3 | 20.72 | 0.92 | 1.92 | 4.94 | 24.97 | 62416 | qPCR |
| 1,000X | 2500 | 2.58 | 24.44 | 0.92 | 1.92 | 1.22 | | 6451 | digital PCR |
| 100X | 2500 | 0.23 | 25.66 | 0.92 | 1.92 | 0.00 | | 576 | digital PCR |
| 10X | 2500 | 0.02 | | | | | | 45 | digital PCR |
| 1X | 2500 | 0.00 | | | | | | 5 | digital PCR |

Table 2 is a detected result of a measuring method for a nucleic acid sample according to an embodiment of the invention. In the present embodiment, the nucleic acid sample to be tested simultaneously contains a wild-type gene with higher concentration and a mutation gene with lower concentration. The object of the present embodiment is to show that the invention can achieve good linear dynamic range when nucleic acid targets with higher concentration and lower concentration both exist.

In Table 2, there are 5 samples to be tested, and each sample to be tested contains both EGFR wild-type and EGFR T790M mutant genes, wherein the mutant has a variant allelic frequency (VAF) of 0.2% to 3.2%. In the present embodiment, two kinds of fluorescent signals are used simultaneously for detection, which are respectively an FAM fluorescent signal used to detect the mutant EGFR gene, and a CY5 fluorescent signal used to detect the wild-type EGFR gene. In the first measurement, the copy number per well measured by the FAM fluorescent signal of digital PCR is 0.006, which is multiplied by a reaction well number of 2500 to obtain a total copy number of 15 for the mutant EGFR. The Cq value measured by CY5 fluorescent signal of quantitative PCR is 28.5. At this time, the Cq value of a single copy number is 30.22, the PCR efficiency is 0.90, and the converted copy number per well is 1.9 to the power of 1.72 (30.22 minus 28.5), i.e., 3.01, which is multiplied by a reaction well number of 2500 to obtain a total copy number of 7516 for the wild-type EGFR. The total copy number of the mutant EGFR is divided by the total copy number of the wild-type EGFR to obtain 0.20% (15/7516) for the VAF measured by the present method, which is very close to the VAF of the sample to be tested. According to this method, the VAF of the other four measurements can be obtained. The VAF of a total of five measurements and the VAF of the dilution ratio of the sample to be tested have a linear regression R² of 0.9996, indicating the feasibility of the method and linearity maintains excellent in the VAF interval at lower concentration (0.2% to 3.2%).

**Table 2**

| Sample EGFR T790M (mutant) dilution ratio | Sample EGFR (wild-type) dilution ratio | Sample VAF (from dilution ratio) | Mutant (FAM) | | | Wild-type (CY5) | | | | | | | Sample VAF measured by present method |
|---|---|---|---|---|---|---|---|---|---|---|---|---|---|
| | | | Copy number /well (from digital PCR) | Cq | Total copy number | Copy number /well (from digital PCR) | Cq | PCR efficiency | Base | Exponent | Converted copy number/ well (base^ exponent) | Total copy number | |
| 1X | 200X | 0.20% | 0.006 | 30.22 | 15 | >3 | 28.50 | 0.90 | 1.90 | 1.72 | 3.01 | 7516 | 0.20% |
| 2X | 200X | 0.40% | 0.012 | 30.14 | 30 | >3 | 28.47 | 0.90 | 1.90 | 1.67 | 2.92 | 7302 | 0.41% |
| 4X | 200X | 0.80 % | 0.027 | 30.13 | 68 | >3 | 28.51 | 0.90 | 1.90 | 1.62 | 2.82 | 7049 | 0.96% |
| 8X | 200X | 1.60% | 0.045 | 30.06 | 113 | >3 | 28.46 | 0.90 | 1.90 | 1.60 | 2.79 | 6981 | 1.61% |
| 16X | 200X | 3.20% | 0.098 | 30.07 | 245 | >3 | 28.42 | 0.90 | 1.90 | 1.65 | 2.88 | 7209 | 3.40% |

Based on the above, the invention provides a measuring method for a nucleic acid sample. The measuring method of the invention is able to successfully detect sample concentration without diluting the sample when nucleic acid sample containing high-concentration nucleic acid target is measured via digital PCR. When the nucleic acid targets in the nucleic acid sample have wide concentration range (especially for clinical samples), it is unnecessary to dilute the nucleic acid sample, so the nucleic acid targets with a higher concentration and a lower concentration can be successfully measured at the same time. In addition, the issue of reduced sensitivity in which the nucleic acid target with a lower concentration is over-diluted can be avoided, so the dynamic range of detection and operational convenience can be effectively improved. In more detail, the nucleic acid target with higher concentration is detected by the digital PCR detection, and the Cq value can be converted into a copy number via the converting step of the invention. As for the nucleic acid target with lower concentration, the copy number of the nucleic acid target in the nucleic acid sample can be directly measured by quantitative PCR.

Moreover, when the nucleic acid targets in the nucleic acid sample have wide concentration ranges (especially for clinical samples), it is unnecessary to dilute the nucleic acid sample, so the nucleic acid targets with higher concentration and lower concentration can be measured successfully at the same time, and the issue of reduced sensitivity in which the nucleic acid target with a lower concentration is over-diluted can be avoided.

## Claims

1. A measuring method for a nucleic acid sample, which simultaneously performs a digital PCR and a quantitative real-time PCR to detect the nucleic acid sample with a high-concentration nucleic acid target and a low-concentration nucleic acid target at one time, wherein a copy number of the high-concentration nucleic acid target in the nucleic acid sample is greater than 10000, and a copy number of the low-concentration nucleic acid target in the nucleic acid sample is 10000 or less, the measuring method for the nucleic acid sample being **characterized in** comprising:
providing a testing plate having a plurality of reaction wells to perform a digital and quantitative real-time PCR on the nucleic acid sample; and
directly quantifying the copy number of the low-concentration nucleic acid target by the digital PCR, and after a Cq value of the high-concentration nucleic acid target is detected by the quantitative real-time PCR, a converting step is performed to obtain the copy number of the high-concentration nucleic acid target in the nucleic acid sample.

2. The measuring method for the nucleic acid sample of claim 1, wherein a PCR efficiency is obtained by a quantitative real-time PCR reaction curve, and then the converting step is performed, the converting step comprising:
adding a number of 1 to the PCR efficiency as a base;
subtracting the Cq value of the high-concentration nucleic acid target from a Cq value of the low-concentration nucleic acid target to obtain ΔCq as an exponent;
using the base and the exponent for a power operation to obtain a copy number of a nucleic acid target of each of the reaction wells; and
multiplying the copy number of the nucleic acid target of each of the reaction wells by a number of the reaction wells in the digital PCR to obtain a total copy number of the high-concentration nucleic acid target.

3. The measuring method for the nucleic acid sample of claim 1, wherein the digital and quantitative real-time PCR is performed using 64 or more reaction wells.

4. The measuring method for the nucleic acid sample of claim 3, wherein the digital and quantitative real-time PCR is performed using 64 to 20000 reaction wells.

5. The measuring method for the nucleic acid sample of claim 1, wherein a dynamic range is increased to 9 logs after the converting step.

6. The measuring method for the nucleic acid sample of claim 1, wherein when all of the reaction wells demonstrate positive responses to a nucleic acid target, the nucleic acid target is the high-concentration nucleic acid target.

7. The measuring method for the nucleic acid sample of claim 1, wherein when not all of the reaction wells demonstrate a positive responses to a nucleic acid target, the nucleic acid target is the low-concentration nucleic acid target, and the copy number of the low-concentration nucleic acid target in the nucleic acid sample is directly measured.

## Patentansprüche

1. Messverfahren für eine Nukleinsäureprobe, das gleichzeitig eine digitale PCR und eine quantitative Echtzeit-PCR durchführt, um die Nukleinsäureprobe mit einem hochkonzentrierten Nukleinsäureziel und einem niedrigkonzentrierten Nukleinsäureziel gleichzeitig nachzuweisen, wobei eine Kopienzahl des hochkonzentrierten Nukleinsäureziels in der Nukleinsäureprobe größer als 10000 ist und eine Kopienzahl des niedrigkonzentrierten Nukleinsäureziels in der Nukleinsäureprobe 10000 oder weniger ist, wobei das Messverfahren für die Nukleinsäureprobe **dadurch gekennzeichnet ist, dass** es umfasst:
Bereitstellen einer Testplatte mit einer Vielzahl von Reaktionsvertiefungen, um eine digitale und quantitative Echtzeit-PCR an der Nukleinsäureprobe durchzuführen; und
direktes Quantifizieren der Kopienzahl des niedrigkonzentrierten Nukleinsäureziels durch die digitale PCR, und nachdem ein Cq-Wert des hochkonzentrierten Nukleinsäureziels durch die quantitative Echtzeit-PCR nachgewiesen ist, wird ein Umwandlungsschritt durchgeführt, um die Kopienzahl des hoch konzentrierten Nukleinsäureziels in der Nukleinsäureprobe zu erhalten.

2. Messverfahren für die Nukleinsäureprobe nach Anspruch 1, wobei eine PCR-Effizienz durch eine quantitative Echtzeit-PCR-Reaktionskurve erhalten wird und dann der Umwandlungsschritt durchgeführt wird, wobei der Umwandlungsschritt umfasst:
Addieren einer Zahl von 1 zu der PCR-Effizienz als eine Basis;
Subtrahieren des Cq-Wertes des hochkonzentrierten Nukleinsäureziels von einem Cq-Wert des niedrigkonzentrierten Nukleinsäureziels, um ΔCq als einen Exponenten zu erhalten;
Verwenden der Basis und des Exponenten für einen Potenzierungsvorgang, um eine Kopienzahl eines Nukleinsäureziels jeder der Reaktionsvertiefungen zu erhalten; und
Multiplizieren der Kopienzahl des Nukleinsäureziels jeder der Reaktionsvertiefungen mit einer Anzahl der Reaktionsvertiefungen in der digitalen PCR, um eine Gesamtkopienzahl des hochkonzentrierten Nukleinsäureziels zu erhalten.

3. Messverfahren für die Nukleinsäureprobe nach Anspruch 1, wobei die digitale und quantitative Echtzeit-PCR unter Verwendung von 64 oder mehr Reaktionsvertiefungen durchgeführt wird.

4. Messverfahren für die Nukleinsäureprobe nach Anspruch 3, wobei die digitale und quantitative Echtzeit-PCR unter Verwendung von 64 bis 20000 Reaktionsvertiefungen durchgeführt wird.

5. Messverfahren für die Nukleinsäureprobe nach Anspruch 1, wobei ein dynamischer Bereich nach dem Umwandlungsschritt auf 9 logs erhöht wird.

6. Messverfahren für die Nukleinsäureprobe nach Anspruch 1, wobei, wenn alle der Reaktionsvertiefungen positive Antworten auf ein Nukleinsäureziel zeigen, das Nukleinsäureziel das hochkonzentrierte Nukleinsäureziel ist.

7. Messverfahren für die Nukleinsäureprobe nach Anspruch 1, wobei, wenn nicht alle der Reaktionsvertiefungen eine positive Antwort auf ein Nukleinsäureziel zeigen, das Nukleinsäureziel das Nukleinsäureziel mit niedriger Konzentration ist, und die Kopienzahl des Nukleinsäureziels mit niedriger Konzentration in der Nukleinsäureprobe direkt gemessen wird.

## Revendications

1. Procédé de mesure pour un échantillon d'acide nucléique, qui réalise simultanément une PCR numérique et une PCR quantitative en temps réel pour détecter en même temps dans l'échantillon d'acide nucléique une cible d'acide nucléique à concentration élevée et une cible d'acide nucléique à concentration faible, dans lequel un nombre de copies de la cible d'acide nucléique à concentration élevée dans l'échantillon d'acide nucléique est supérieur à 10 000, et un nombre de copies de la cible d'acide nucléique à concentration faible dans l'échantillon d'acide nucléique est de 10 000 ou moins, le procédé de mesure pour l'échantillon d'acide nucléique étant **caractérisé en ce qu'**il comprend :
la fourniture d'une plaque de test ayant une pluralité de puits de réaction pour réaliser une PCR numérique et quantitative en temps réel sur l'échantillon d'acide nucléique ; et
la quantification directe du nombre de copies de la cible d'acide nucléique à concentration faible par PCR numérique, et après la détection d'une valeur de Cq de la cible d'acide nucléique à concentration élevée par la PCR quantitative en temps réel, une étape de conversion est réalisée pour obtenir le nombre de copies de la cible d'acide nucléique à concentration élevée dans l'échantillon d'acide nucléique.

2. Procédé de mesure pour l'échantillon d'acide nucléique selon la revendication 1, dans lequel une efficacité de PCR est obtenue par une courbe de réaction de PCR quantitative en temps réel, puis l'étape de conversion est réalisée, l'étape de conversion comprenant :
l'ajout d'un nombre de 1 à l'efficacité de PCR en tant que base ;
la soustraction de la valeur de Cq de la cible d'acide nucléique à concentration élevée d'une valeur Cq de la cible d'acide nucléique à concentration faible pour obtenir ΔCq sous la forme d'un exposant ;
l'utilisation de la base et de l'exposant pour une opération de puissance pour obtenir un nombre de copies d'une cible d'acide nucléique de chacun des puits de réaction ; et
la multiplication du nombre de copies de la cible d'acide nucléique de chaque puits de réaction par un nombre des puits de réaction dans la PCR numérique pour obtenir un nombre de copies total de la cible d'acide nucléique à concentration élevée.

3. Procédé de mesure pour l'échantillon d'acide nucléique selon la revendication 1, dans lequel la PCR numérique et quantitative en temps réel est réalisée en utilisant 64 puits de réaction ou plus.

4. Procédé de mesure pour l'échantillon d'acide nucléique selon la revendication 3, dans lequel la PCR numérique et quantitative en temps réel est réalisée en utilisant de 64 à 20 000 puits de réaction.

5. Procédé de mesure pour l'échantillon d'acide nucléique selon la revendication 1, dans lequel une plage dynamique est portée à 9 logs après l'étape de conversion.

6. Procédé de mesure pour l'échantillon d'acide nucléique selon la revendication 1, dans lequel lorsque tous les puits de réaction présentent des réponses positives à une cible d'acide nucléique, la cible d'acide nucléique est la cible d'acide nucléique à concentration élevée.

7. Procédé de mesure pour l'échantillon d'acide nucléique selon la revendication 1, dans lequel lorsque tous les puits de réaction ne présentent pas une réponse positive à une cible d'acide nucléique, la cible d'acide nucléique est la cible d'acide nucléique à concentration faible, et le nombre de copies de la cible d'acide nucléique à concentration faible dans l'échantillon d'acide nucléique est directement mesuré.
